# EUROPEAN PATENT APPLICATION

(11) **EP 4 643 817 A2**
(43) Date of publication of application: **05.11.2025**
(21) Application number: 25202876.6
(22) Date of filing: 06.04.2021
(51) Int. Cl.: A61F 2/07

(54) **TWO-STAGE DEPLOYMENT SHEATH SYSTEMS AND METHODS**

(62) Divisional of application: 21721749.6
(71) Applicant: W. L. Gore & Associates, Inc., Newark, DE 19711 (US)
(72) Inventor: HONEYFIELD, Evan, Newark, 19711 (US)
(74) Representative: HGF

(57) **Abstract**

An implantable medical device including an expandable device having a longitudinal length and operable to be compressed to a first diameter and deployed to a second diameter, wherein the second diameter is greater than the first diameter, and wherein a first portion that is less than the longitudinal length of the expandable device and a second portion that is less than the longitudinal length of the expandable device are defined on the expandable device. The medical device includes a first constraining member operable to constrain the first portion of the expandable device at the first diameter; a second constraining member operable to constrain the second portion of the expandable device at the first diameter.

## Description

### FIELD

The present disclosure relates generally to apparatuses, systems, and methods that include coverings used in delivery of implantable medical devices. More specifically, the disclosure relates to apparatuses, systems, and methods that include coverings for constraining expandable, implantable medical devices during device delivery.

### BACKGROUND

Minimally invasive delivery techniques for implantable medical devices have a variety of advantages, such as reduced trauma, risk of infection, and recovery time. Examples of implantable medical devices include stents and stent-grafts utilized to radially support, treat and / or otherwise augment tubular passages in the body, including arteries, veins, airways, gastrointestinal tracts, and biliary tracts. Additional examples of implantable medical devices include prosthetic valves (e.g., prosthetic heart valves). Transcatheter delivery is a technique for delivering such implantable medical devices, where the medical device to be delivered begins in a diametrically compressed state for delivery and then is expanded (e.g., self-expanding or manually expandable) at a treatment site in the body of a patient.

Stents, stent-grafts, prosthetic valves, filters, and other implantables may be deployed by being plastically deformed (e.g., using an inflatable balloon) or permitted to self-expand and elastically recover from a collapsed or constrained, delivery diameter to an expanded, deployed diameter.

For example, U.S. Patent 6,224,627, entitled "Remotely removable covering and support," filed June 15, 1998, describes, among other things, a thin tubular multiple filament (film or fiber) structure that can hold high internal pressures. When desired, an extension of the filaments can be pulled in any direction to unfurl the structure. The structure can be useful for constraining and deploying self-expanding stent or stent graft delivery systems, balloon dilatation catheters, removable guide wire lumens for catheters, drug infusion or suction catheters, guide wire bundling casings, removable filters, removable wire insulation, removable packaging and other applications.

### SUMMARY

Various disclosed concepts relate to an implantable medical device. According to one example ("Example 1"), the implantable medical device includes an expandable device having a longitudinal length and operable to be compressed to a first diameter and deployed to a second diameter, wherein the second diameter is greater than the first diameter, and wherein a first portion that is less than the longitudinal length of the expandable device and a second portion that is less than the longitudinal length of the expandable device are defined on the expandable device, a first constraining member operable to constrain the first portion of the expandable device at the first diameter; and a second constraining member operable to constrain the second portion of the expandable device at the first diameter.

According to another example further to Example 1 ("Example 2"), the first constraining member defines a first release zone and the second constraining member defines a second release zone.

According to another example further to Example 2 ("Example 3"), the first constraining member further comprises a first deployment line, wherein the first deployment line is configured to release the first constraining member along the first release zone by tensioning the first deployment line, and wherein the second constraining member further comprises a second deployment line, wherein the second deployment line is configured to release the second constraining member along the second release zone by tensioning the second deployment line.

According to another example further to Example 3 ("Example 4"), the first release zone is configured to release when a first tension force is applied across the first deployment line, wherein the second release zone is configured to release when a second tension force is applied across the second deployment line, and wherein the first tension force is higher than the second tension force.

According to another example further to Example 3 ("Example 5"), the first release zone is configured to release when a first tension force is applied across the first deployment line, wherein the second release zone is configured to release when a second tension force is applied across the second deployment line, and wherein the first tension force is lower than the second tension force.

According to another example further to any of the preceding Examples ("Example 6"), the second constraining member extends over at least part of the first portion of the expandable member.

According to another example further to Example 6 ("Example 7"), the second constraining member is positioned between the first constraining member and the expandable member where the second constraining member extends over at least part of the first portion of the expandable member.

According to another example further to Example 6 ("Example 8"), the second constraining member is disposed exterior to the first constraining member and the expandable member where the second constraining member extends over at least part of the first portion of the expandable member.

According to another example further to any of the preceding Examples ("Example 9"), the second constraining member includes an everted portion.

According to another example further to Example 9 ("Example 10"), the everted portion of the second constraining member is disposed about at least part of the first portion expandable member and the first constraining member.

According to another example further to Example 10 ("Example 11"), the everted portion of the second constraining member is disposed along the longitudinal length of the expandable member.

According to another example further to any of the preceding Examples ("Example 12"),the first constraining member comprises a first knit sleeve comprising a first plurality of strands.

According to another example further to any of the preceding Examples ("Example 13"), the second constraining member comprises a second knit sleeve comprising a second plurality of strands.

According to another example further to Examples 12 or 13 ("Example 14"), the first knit sleeve and the second knit sleeve are knitted.

According to another example further to any of the preceding Examples ("Example 15"), the first constraining member and the second constraining member are warp knit.

According to another example further to any of the preceding Examples ("Example 16"),the first constraining member is operable to release after the second constraining member has been released.

According to another example further to any one of Examples 3 to 16 ("Example 17"), the first deployment line is engaged with the second constraining member such that when the second constraining member is released, the first deployment line is accessible to activate release of the first constraining member.

According to another example further to Example 17 ("Example 18"), the first deployment line is coupled to the second constraining member such that when the second constraining member has translated away from the expandable member, the first deployment line is tensioned and activates release of the first constraining member.

According to another example further to any one of Examples 12 to 18 ("Example 19"), the first plurality of strands and second plurality of strands include matching strand properties, including any one of strand thickness, strand denier, strand coefficient of friction, strand material, strand coatings, strand treatments, strand stiffness, or any combination thereof.

According to another example further to any one of Examples 12 to 18 ("Example 20"), the first plurality of strands and second plurality of strands include differing strand properties, including any one of strand thickness, strand denier, strand coefficient of friction, strand material, strand coatings, strand treatments, strand stiffness, or any combination thereof.

According to another example further to any one of Examples 12 to 20 ("Example 21"), the second plurality of strands extend from the first plurality of strands.

According to one example ("Example 22"), an implantable medical device is included. The implantable medical device has an expandable member having a proximal end and a distal end, wherein the expandable member has a longitudinal length defined between the proximal end and the distal end; a first constraining member disposed about the expandable member at a first portion that is less than all of the longitudinal length of the expandable member, wherein the first constraining member maintains the expandable member at the first portion at a constrained diameter; and a second constraining member including a first segment and an everted segment, wherein the first segment of the second constraining member is disposed about the expandable member at a stabilizing portion that is a remainder of the longitudinal length where the first constraining member is not disposed about the expandable member, wherein the second constraining member maintains the expandable member at the stabilizing portion at the constrained diameter, wherein the everted segment of the second constraining member is disposed about the first segment of the second constraining member and the stabilizing portion of the expandable member.

According to one example further to Example 22 ("Example 23"), the first constraining member further comprises a first release zone and a first deployment line, and wherein the second constraining member further comprises a second release zone and a second deployment line.

According to one example further to Example 23 ("Example 24"), the first deployment line is operable to release the first release zone when at least a first deployment force is applied to the first deployment line, and wherein the second deployment line is operable to release the second release zone when at least a second deployment force is applied to the second deployment line.

According to one example further to Example 24 ("Example 25"), the first deployment force is greater than the second deployment force.

According to one example further to Example 25 ("Example 26"), the first deployment force is less than the second deployment force.

According to another example further to any one of Examples 22 to 26 ("Example 27"), the first constraining member comprises a first plurality of warp-knit strands.

According to another example further to any one of Examples 22 to 27 ("Example 28"), the second constraining member comprises a second plurality of warp-knit strands.

According to another example further to any one of Examples 22 to 28 ("Example 29"), the everted segment of the second constraining member is disposed about the first constraining member.

According to another example further to any one of Examples 22 to 29 ("Example 30"), the stabilizing portion of the expandable member is operable to deploy to a second diameter when the second constraining member is removed from the expandable member.

According to another example further to any one of Examples 22 to 30 ("Example 31"), the first deployment line is engaged with the second constraining member such that first constraining member is operable to be released after the second constraining member has been released and the stabilizing portion of the expandable member has been deployed.

According to another example further to any one of Examples 22 to 31 ("Example 32"), the first portion of the expandable member extends to the proximal end of the expandable member.

According to another example further to any one of Examples 22 to 32 ("Example 33"), the stabilizing portion of the expandable member extends to the distal end of the expandable member.

According to another example further to any one of Examples 22 to 33 ("Example 34"), the second sheath is configured to release such that the proximal end of the expandable member is deployed first.

According to another example further to any one of Examples 22 to 34 ("Example 35"), the first sheath is configured to release after the second sheath is released, such that the proximal end of the expandable member is deployed subsequent to the distal end being deployed.

According to one example ("Example 36"), a method of manufacturing an implantable medical device is included, the method comprising compressing an expandable member radially inward; knitting a first plurality of strands to form a first constraining member with a first deployment line; installing the first constraining member on a first portion of the expandable member; knitting a second plurality of strands to form a second constraining member; installing the second constraining member on a second portion of the expandable member; and everting a segment of the second constraining member back over the second portion of the expandable member.

According to another example further to Examples 36 ("Example 37"), the method further comprises initiating release of a first release zone of the first constraining member and a second release zone of the second constraining member.

According to another example further to Examples 37 ("Example 38"), the method further comprises partially releasing the first release zone until a first end of the first release zone is positioned proximate a proximal end of the expandable member.

According to another example further to Examples 38 ("Example 39"), the method further comprises partially releasing the second release zone until a second end of the second release zone is positioned proximate the proximal end of the expandable member.

According to one example ("Example 40"), a method of deploying an implantable medical device is included, the method comprising positioning an expandable member in a lumen of a patient, wherein a first portion of the implantable medical device is constrained by a first constraining member in a compressed configuration and a second portion of the expandable member is constrained by a second constraining member in the compressed configuration; removing the second constraining member from the second portion of the expandable member; removing the first constraining member from the first portion of the expandable member.

According to another example further to Examples 40 ("Example 41"), the method further comprises retaining a free end of a second deployment line remote from the expandable medical device, wherein the second deployment line is engaged with a second release zone of the second constraining member that is configured to disengage the second constraining member from the expandable member; and applying sufficient force to the free end of the second deployment line to activate the second release zone.

According to another example further to Examples 41 ("Example 42"), the method further comprises applying sufficient force to a free end of a first deployment line to activate a release zone of the first constraining member, wherein the first deployment line is engaged with the release zone of the first constraining member, wherein the release zone of the first constraining member is configured to disengage the first constraining member from the expandable member.

The foregoing Examples are just that, and should not be read to limit or otherwise narrow the scope of any of the inventive concepts otherwise provided by the instant disclosure. While multiple examples are disclosed, still other embodiments will become apparent to those skilled in the art from the following detailed description, which shows and describes illustrative examples. Accordingly, the drawings and detailed description are to be regarded as illustrative in nature rather than restrictive in nature.

### BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.
FIG. 1 is a medical device delivery system in accordance with an embodiment.
FIG. 2 is an expandable member constrained at a first diameter in accordance with an embodiment.
FIG. 3 is a sectional view of a removable constrain in accordance with an embodiment.
FIG. 4a is a sectional view of FIG. 4b, which is a removable constraint constraining a device in accordance with an embodiment.
FIG. 5a is a sectional view of FIG. 5b, which is a removable constraint constraining a device, where an exterior layer of the removable constraint has been partially released, in accordance with an embodiment.
FIG. 6a is a sectional view of FIG. 6b, which is a removable constraint constraining a device, where an exterior layer of the removable constraint has been partially released such that a second segment of the removable constraint is exposed, in accordance with an embodiment.
FIG. 7a is a sectional view of FIG. 7b, which is a removable constraint constraining a device, where an exterior layer of the removable constraint has been partially released such that a second segment of the removable constraint is exposed and an interior layer of a first segment is exposed, in accordance with an embodiment.
FIG. 8a is a sectional view of FIG. 8b, which is a removable constraint constraining a device, where an exterior layer of the removable constraint has been fully released such that a second segment of the removable constraint is exposed and an interior layer of a first segment is fully exposed and partially released such that the device is partially unconstrained, in accordance with an embodiment.
FIG. 9a is a sectional view of FIG. 9b, which is a removable constraint constraining a device, where an exterior layer of the removable constraint has been fully released, a first segment is fully released, and a portion of the second segment is partially released such that the device is partially unconstrained, in accordance with an embodiment.
FIG. 10a is a sectional view of FIG. 10b, which is a removable constraint constraining a device, where an exterior layer of the removable constraint has been fully released, a first segment is fully released, and a portion of the second segment is partially released such that the device is partially unconstrained and nearly fully-deployed, in accordance with an embodiment.
FIG. 11a is a sectional view of FIG. 11b, which is a removable constraint fully released from a device such that the device is in an unconstrained configuration, in accordance with an embodiment.
FIG. 12 is a sectional view of a removable constraint having two segments with one segment partially everted over itself, in accordance with an embodiment.
FIG. 13 is a sectional view of a removable constraint having two segments with one segment including a deployment line coupled to or extending from a portion of the other segment, in accordance with an embodiment.
FIG. 14 is a sectional view of a removable constraint having two segments where the first segment is aligned coaxially with the device along a portion of the length of the device and the second segment is aligned coaxially along the full length of the device, in accordance with an embodiment.
FIG. 15 is a sectional view of a removable constraint having two segments with varying properties, in accordance with an embodiment.
FIG. 16 is a sectional view of a removable constraint having two segments with varying properties, one of the segments surrounding the entire length of a device and everting onto a portion of itself, in accordance with an embodiment.

### DETAILED DESCRIPTION

### Definitions and Terminology

This disclosure is not meant to be read in a restrictive manner. For example, the terminology used in the application should be read broadly in the context of the meaning those in the field would attribute such terminology.

With respect to terminology of inexactitude, the terms "about" and "approximately" may be used, interchangeably, to refer to a measurement that includes the stated measurement and that also includes any measurements that are reasonably close to the stated measurement. Measurements that are reasonably close to the stated measurement deviate from the stated measurement by a reasonably small amount as understood and readily ascertained by individuals having ordinary skill in the relevant arts. Such deviations may be attributable to measurement error, differences in measurement and/or manufacturing equipment calibration, human error in reading and/or setting measurements, minor adjustments made to optimize performance and/or structural parameters in view of differences in measurements associated with other components, particular implementation scenarios, imprecise adjustment and/or manipulation of objects by a person or machine, and/or the like, for example. In the event it is determined that individuals having ordinary skill in the relevant arts would not readily ascertain values for such reasonably small differences, the terms "about" and "approximately" can be understood to mean plus or minus 10% of the stated value.

Outward radial expansion force or radial force generally refers to the force caused by a device acting on a removable constraint at any point during deployment of the device. The radial force may be a result of a self-expanding member. Such self-expanding members may include shape memory alloys which exert an outward radial expansion force when compressed and / or constrained. Outward radial expansion force may also refer to other forces causing a device or member to expand radially outward such as inflation of an angioplastic balloon.

Constraining force generally refers to the force a constraining member is resisting when constraining a device, in some embodiments a self-expanding device. The constraining force may be considered a normal force against the outward radial expansion force. The constraining force may be limited in some embodiments to a threshold amount until the constraining member is no longer able to resist the outward radial expansion force of a self-expanding device, at which point the medical device may deploy due to the outward radial expansion force overcoming the constraining force of the constraining member causing the constraining member to disengage.

Deployment force generally refers to the force required to deploy the medical device by disengaging the constraining member. In some embodiments the deployment force is the force required to tension an activation line of a constraining member in order to activate disengagement of the constraining member from the medical device

For reference, the term "circumference" is not meant to require a circular cross-section, and is instead to be understood broadly to reference an outer surface or dimension of the removable constraint.

### Description of Various Embodiments

Persons skilled in the art will readily appreciate that various aspects of the present disclosure can be realized by any number of methods and apparatuses configured to perform the intended functions. It should also be noted that the accompanying drawing figures referred to herein are not necessarily drawn to scale, but may be exaggerated to illustrate various aspects of the present disclosure, and in that regard, the drawing figures should not be construed as limiting.

The disclosed devices herein may provide the ability to implement a controlled deployment of expanding and self-expanding devices. The constraints discussed herein include, but are not limited to, features allowing for precise positioning of a device during deployment. The constraints (e.g., knitted constraints) can provide sufficient constraining force to maintain a self-expanding device in a constrained configuration while also releasing the self-expanding device by applying low-levels of force so as to minimize displacement of the device from the desired deployment position. The constraint may also permit variable forces required for releasing the removable constraint from the device which can permit the expandable device to partially deploy at the desired deployment position and to act as an anchor at that position. The descriptions of the systems, devices, and methods included below describe these and other objects of the present disclosure.

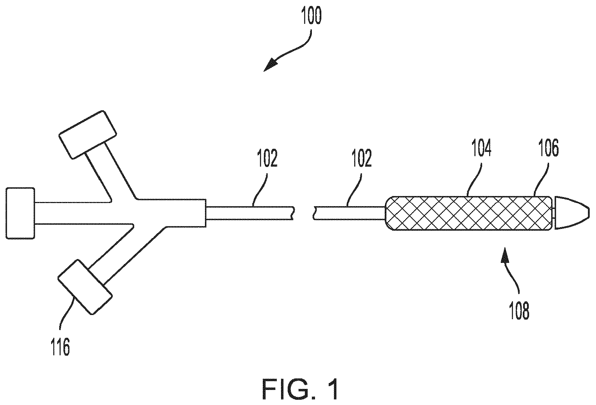

The system shown in FIG. 1 is provided as an example of the various features of the system and, although the combination of those illustrated features is clearly within the scope of invention, that example and its illustration is not meant to suggest the inventive concepts provided herein are limited from fewer features, additional features, or alternative features to one or more of those features shown in FIG. 1 is a plan view of a delivery system 100 including a catheter 102 with a removable constraint 104, according to some embodiments. As shown in FIG. 1, the removable constraint 104 is configured to constrain an implantable medical device 106 to a delivery configuration. The removable constraint 104 may include one or more fibers or strands 108 arranged about the device 106 to maintain the device 106 and the removable constraint 104 in a constrained or delivery configuration. Removeable constraints 104 may include various configurations and constructions such as discussed in US Pat. No. 6,224,627 by Armstrong and issued May 1, 2001, which is hereby incorporated by reference in its entirety and for all purposes.

The removable constraint 104 is coaxially arranged along a length of the device 106. The removable constraint 104 is also circumferentially arranged about the device 106 and may substantially cover the device 106 for delivery. The one or more strands 108 may be arranged within a lumen (not shown) of the catheter 102 and extend toward a proximal end of the catheter 102, which may, in turn, be arranged external to a patient during delivery of the device 106. The one or more strands 108 may include a proximal end that a user may tension in order to release the removable constraint 104 and deploy the device 106.

In certain instances, the one or more strands 108 release such that interlocking portions (e.g., overlapping fibers or knits) sequentially release along the length of the device 106. As is explained in greater detail below, the removable constraint 104 is formed by interlocking together the one or more strands 108 on the device 106. The one or more strands 108 may form release zones or releasing the device 106. The device 106 may be a stent, stent-graft, a balloon, prosthetic valve, filter, anastomosis device, occluder or a similar device.

The system shown in FIG.1 is provided as an example of the various features of the system. Although the combination of those illustrated features is clearly within the scope of invention, the example shown in FIG. 1 and its illustration is not meant to suggest the inventive concepts provided herein are limited from fewer features, additional features, or alternative features to one or more of those features shown in the figures.

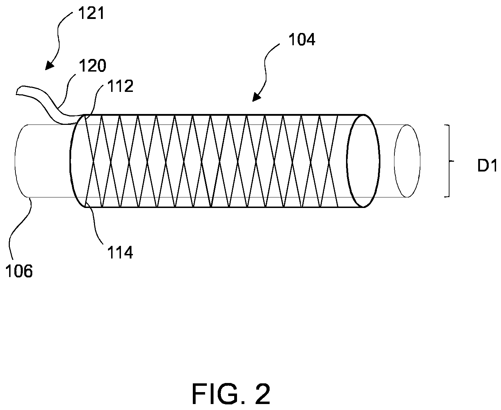

FIG. 2 is a side view of the device 106 including the removable constraint 104, in accordance with an embodiment. As shown, the device 106 includes a delivery diameter D1 and a deployed diameter D2 (not shown) that is larger than the delivery diameter D1. The removable constraint 104 is arranged about the device 106 at the delivery diameter D1. When the removable constraint 104 is removed from the device 106, the device expands to a deployed diameter D2. In some embodiments, the deployed diameter D2 is the diameter of the device 106 when unconstrained. In other embodiments, the deployed diameter D2 is the diameter of the device 106 once the device 106 has been delivered to a target site and has engaged with the lumen wall at the target site.

The device 106 may have a desired deployed diameter D2 from about 5mm to about 15mm, or from about 6mm to about 9mm, or from about 6mm to about 12mm, for example, and a delivery diameter D1 that is less than the deployed diameter D2. For example, in some instances, a ratio of the delivery diameter D1 of the device 106 to the deployed diameter D2 (not shown) of the device 106 is less than about 0.3, less than about 0.29, less than about 0.28, less than about 0.27, or less than about 0.26.

As shown in FIGs. 1 and 2, the removable constraint 104 includes at least two interlocking strands 108 in the form of a warp knit. For example, the removable constraint 104 may include a first interlocking strand 112 and a second interlocking strand 114. The first and / or the second interlocking strand(s) 112, 114 may operate, for example, as a first deployment line 120. The first deployment line 120 may be configured to release the removable constraint 104 and release the device 106 from the delivery diameter D1 to the deployed diameter D2 in response to a deployment force applied to the first deployment line 120. It is within the scope of this disclosure to form a removable constraint with two, four, six, eight, or any even number of interlocking strands or any odd number of interlocking strands.

The device 106 has a radial force at the delivery diameter D1. The radial force generally refers to the force caused by the device 106 acting on the removable constraint 104 at any point during deployment of the device 106. As discussed above, the interlocking strands 112, 114 are adapted to be removed with a deployment force applied to the deployment line 120. The radial force of the device 106 may be compared to the deployment force for removing the removable constraint 104 from the device 106 as a ratio. In some instances, the ratio of this radial force of the device 106 to the deployment force applied to the deployment line 120 is less than about 500:1. In other instances, the ratio of this radial force of the device 106 to the deployment force applied to the deployment lines 120 is less than about 475. In addition, the ratio of this radial force of the device 106 to the deployment force applied to the deployment lines 120 may be less than about 450. In addition, the ratio of this radial force of the device 106 to the deployment force applied to the deployment lines 120 is less than about 425 in other instances. Further, the ratio of the radial force to the deployment force may be between about .01 and 7000 (or any value therebetween)

It will be understood that the deployment force of the device is the force applied to the deployment line 120 of the removable constraint 104 at or near the position where the deployment line 120 and the body of the removable constraint 104 interface and sufficient to effectuate at least partial deployment of the device 106. Because, in some embodiments, the delivery system 100 may affect how much force a user must apply to the deployment line 120 (e.g., frictional forces, pully or gears, etc.), deployment force for the purposes of the discussion relate to the force applied proximate the device 106. Furthermore, in some embodiments, the deployment force may be varied based on the device 106 being constrained. For example, when the device 106 includes high radial forces when constrained at a specific diameter, the radial force of the device 106 may decrease the deployment force necessary to remove the removable constraint 104. In some embodiments, the radial force may be such that the removable constraint 104 may experience accelerated deployment due to the radial force overcoming the removable constraint 104. Although accelerated deployment is generally not desirable, in some embodiments, accelerated deployment may be advantageous. For example, accelerated deployment may be desirable to quickly deploy a portion of the device 106 such that the deployed portion of the device 106 may act as an anchor while deploying the remained of the device 106. These concepts will be discussed in greater detail hereafter. It will also be understood that the deployment force of a removable constraint 104 may vary across the device 106 depending on the amount of radial force transmitted to the removable constrain 104.

The one or more strands 108, which may include the interlocking strands 112, 114 in some embodiments, may be formed of various materials, including, for example, polytetrafluoroethylene (PTFE), expanded polytetrafluoroethylene (ePTFE), polyester, polyurethane, fluoropolymers, such as perfluoroelastomers and the like, polytetrafluoroethylene, silicones, urethanes, ultra-high molecular weight polyethylene, aramid fibers, and combinations thereof. Other embodiments for the interlocking strands 112, 114 can include high strength polymer fibers such as ultra-high molecular weight polyethylene fibers (e.g., Spectra^{®}, Dyneema Purity^{®}, etc.) or aramid fibers (e.g., Technora^{®}, etc.). Generally, any of the foregoing properties may be assessed using ASTM or other recognized measurement techniques and standards, as would be appreciated by a person of ordinary skill in the field.

The one or more strands 108 may be selected to have specific properties such as strand thickness, strand denier, strand coefficient of friction, strand material, strand treatments, strand coatings, and strand stiffness. Similar to the differing diameter, use of differing strand materials for the strands 108 may increase friction between the first and second interlocking strands 108 to help maintain the device 106 in the delivery configuration. Each of the various strands may be selected to include the same strand properties or different strand properties based on the application in which the removable constraint will be used. It will be recognized that the properties of the strands 108 may also be altered by treatments, configurations, and alterations, in addition to material selection. For example, the strands may include fillers or core materials, may be surface treated by etching, vapor deposition, or coronal or other plasma treatment, among other treatment types, including being coated with suitable coating materials.

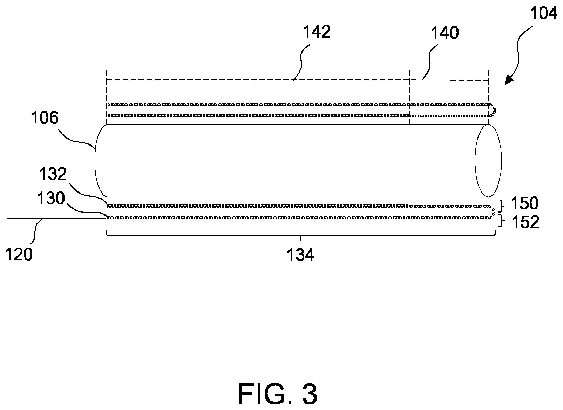

Referring now to FIG. 3, the device 106 may be constrained by a removable constraint 104. The removable constraint 104 may comprise two sleeves and / or portions which include a first segment 130 and a second segment 132. The first segment 130 may include a first set of properties and the second segment 132 may include a second set of properties. It will be noted that at least some of the properties discussed with regards to the first and second segments 130, 132 are different, while some of the properties may be shared. For example, properties that may be shared or different between the first and second segments 130,132 may include thickness, denier, stand coefficient of friction, material, treatments, coatings, and stiffness. For example, in some embodiments the first segment 130 of the removable constraint 104 may include a first material having a first coefficient of friction, whereas the second segment 132 may include a second material having a second coefficient of friction, wherein the first coefficient of friction is higher than the second coefficient of friction.

In some embodiments, the first segment 130 may have differing properties from the second segment 132 to facilitate various functionalities. For example, the first segment 130 may incorporate certain properties in the materials which promote higher constraining force of a device 106. As a result of increased constraining force of the device 106 by the first segment, in some embodiments, the deployment force may also be increased. This may result in difficulty in precisely deploying the device 106 within the lumen of a patient. Thus, in some embodiment, the second segment 132 may incorporate properties in the materials which promote certain characteristics such as a lower deployment force to be applied to the second segment 132 in order to release the second segment 132 from the device 106 in order for the device 106 to deploy. Various examples and combinations of the properties may be implemented on the first and second segments 130, 132 in order to facilitate improved preparation, delivery, and deployment of the device 106. These examples are not to be construed as limiting, but any number of combinations are within the scope of the disclosure.

In some embodiments, the removable constraint 104 includes an axially interior layer 150 and an axially exterior layer 152. The axially interior layer 150 may be positioned against or proximate the device 106. In some embodiments, there may be various elements positioned between the axially interior layer 150 of the removable constraint 104; for example, films and wraps, among others. However, relative to the axially exterior layer 152, the axially interior layer 150 more immediately surrounds the device 106. The removable constraint having axially interior layer 150 and the axially exterior layer 152 may include two sleeves coaxially positioned or, in other embodiments, the layers 150, 152 may be formed by everting one of the first or second segments 130, 132 of the removable constraint.

In some embodiments the first and / or second segment 130, 132 of the removable constraint 104 may include an everted section 134. For example, the first segment 130 may be everted such that the everted section 134 of the second segment 132 is positioned coaxially with the device 106. By including an everted portion 134, the device 106 may be constrained along at least portions of the longitudinal length by two layers of the removable constraint 104. In some embodiments, the everted portion 134 may extend across the entire longitudinal length of the device 106, as seen in FIG. 3.

FIG. 3 shows a device 106 and a removable constraint 104 for treating tissue in a body of a patient, according to some examples. In some embodiments, the device 106 may include various features. The device 106 may be represented by a first portion 140 and a second portion 142. The first and second portions 140, 142 of the device 106 may be defined by the first and second segments 130, 132 of the removable constraint 104. For example, the first portion 140 of the device 106 may correspond to the first segment 130 of the removable constraint 104 and the second portion 142 of the device may correspond to the second segment 132 of the removable constraint 104. In those embodiments where the first segment 130 is everted, the first portion 140 of the device 106 may be defined by the non-everted section of the first segment 130.

The first portion 140 of the device, which in some embodiments is defined by the length of the non-everted section of the first segment 130 of the removable constraint 104, may represent from about 5% to about 95% of the longitudinal length of the device 106. In some embodiments, the first portion represents about 5% of the longitudinal length of the device 106. In some embodiments, the first portion represents about 10% of the longitudinal length of the device 106. In some embodiments, the first portion represents about 15% of the longitudinal length of the device 106. In some embodiments, the first portion represents about 20% of the longitudinal length of the device 106. In some embodiments, the first portion represents about 25% of the longitudinal length of the device 106. In some embodiments, the first portion represents about 30% of the longitudinal length of the device 106. In some embodiments, the first portion represents about 35% of the longitudinal length of the device 106. In some embodiments, the first portion represents about 40% of the longitudinal length of the device 106. In some embodiments, the first portion represents about 45% of the longitudinal length of the device 106. In some embodiments, the first portion represents about 50% of the longitudinal length of the device 106. In some embodiments, the first portion represents about 55% of the longitudinal length of the device 106. In some embodiments, the first portion represents about 60% of the longitudinal length of the device 106. In some embodiments, the first portion represents about 65% of the longitudinal length of the device 106. In some embodiments, the first portion represents about 70% of the longitudinal length of the device 106. In some embodiments, the first portion represents about 75% of the longitudinal length of the device 106. In some embodiments, the first portion represents about 80% of the longitudinal length of the device 106. In some embodiments, the first portion represents about 85% of the longitudinal length of the device 106. In some embodiments, the first portion represents about 90% of the longitudinal length of the device 106. In some embodiments, the first portion represents about 95% of the longitudinal length of the device 106.

Examples of the system 100 discussed herein being implemented are provided. In some embodiments including first and second segments 130, 132 of a removable constraint, the first portion 140 of the device 106 may be deployed prior to deployment of the second portion 142 of the device 106. The first segment 130 of the removable constraint 104 may be actuated such that the first segment 130 releases the first portion 140 of the device 106. For example, the first segment 130 of the removable constraint 104 may have a deployment line 120 extending therefrom. The deployment line 120 may be actuated to initiate or resume release of the removable constraint 104 from the device 106. As the deployment line 120 continues to be actuated, the removable constraint 104 is sequentially released along the length of the device 106. As seen in FIG. 3, an embodiment includes a first segment 130 having an everted portion 134 that is at least partially coaxial with the second segment 132. As the everted portion 134 of the first segment 130 is deployed, the device 106 remains constrained by the second segment 132 of the removable constraint 104 and those portions of the first segment 130 that have not been released. As the everted portion 134 of the first segment 130 releases, the remainder of the removable constraint 104 is exposed from the beneath the everted portion 134.

Once the everted portion 134 of the first segment 130 has been fully released via actuation of the deployment line 120, and as the deployment line 120 continues to be actuated, a portion of the removable constraint 104 that was radially inward from the everted portion 134 (in some embodiments, a portion of the axially interior layer 150) begins to sequentially release. As the radially inward portion of the removable constraint 104 is released, the device 106 becomes unconstrained in those areas where the radially inward portions of the removable constraint was previously constraining the device 106. In those embodiments in which the device 106 is deployable either by self-deployment or by way of an additional device (e.g., a balloon), the device 106 is freed from the removable constraint 104 and is able to further deploy or to be deployed. For example, in those embodiments in which the device 106 is a self-expanding member, at those portions of the device 106 which have been released from the removable constraint 104 are able to expand to the second, larger diameter D2.

The deployment line 120 may continue to be actuated until the removable constraint 104 is fully released from the device 106. The removable constraint 104 may include a separate deployment line for each of the segments 130, 132 of the removable constraint 104. For example, the first segment 130 may include a first deployment line 120a and the second segment may include a second deployment line 120b. In some embodiments, the second deployment line 120b remains inaccessible until the first segment 130 has been released or at least partially released from the device 106.

For example, in one embodiment, the second deployment line 120b is positioned such that it is concealed until a sufficient portion of the first segment 130 is released. The second deployment line 120b may be concealed interior to the first segment 130, within the catheter 102, within the first deployment line 120a, or in another location. In some embodiments, the second deployment line 120b may be coupled to or extend from the first segment 130 such that deployment of the second segment 132 is initiated by the translation of the first segment 130 away from the device 106 via actuation of the first deployment line 120a. The second segment 132 may be released after the first segment 130 has been fully released from the device 106. In some embodiments, it may be understood that at least a portion of the first segment 130, once released, acts as the second deployment line 120b for the second segment 132.

### Example Device and Removable Constraint

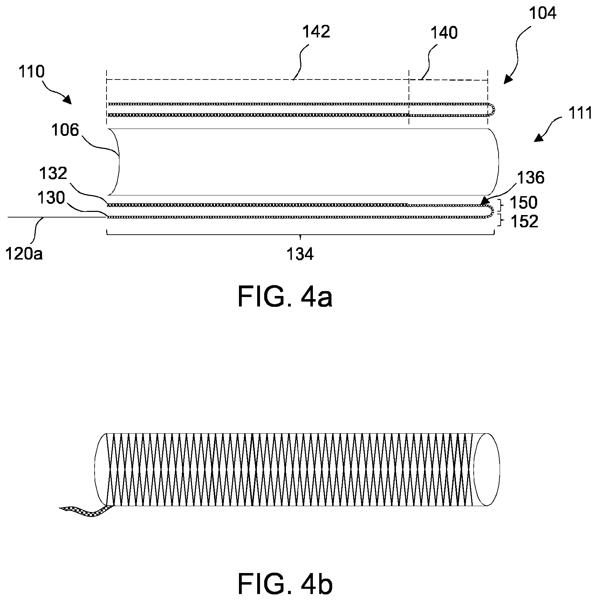

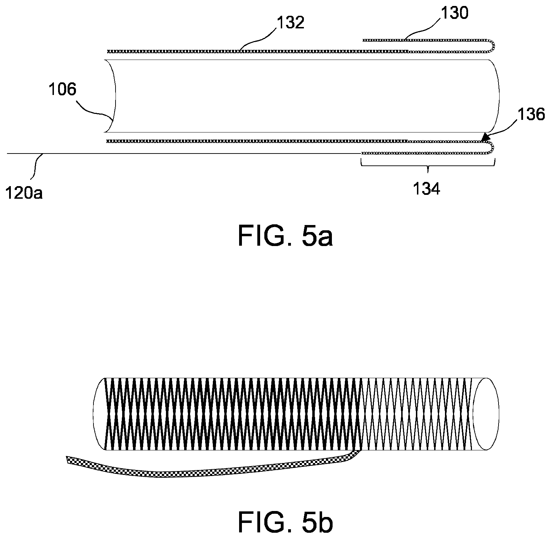

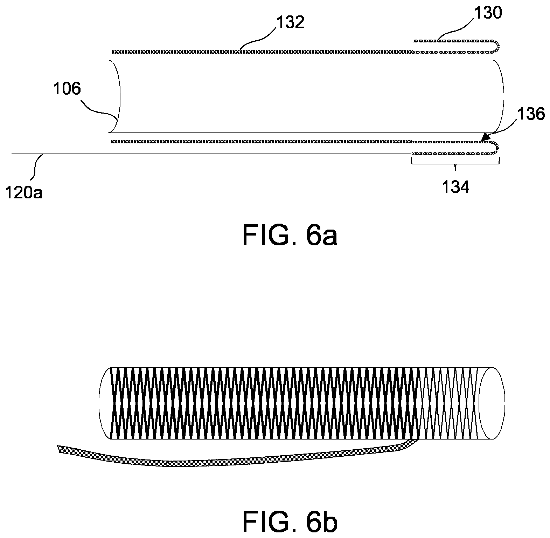

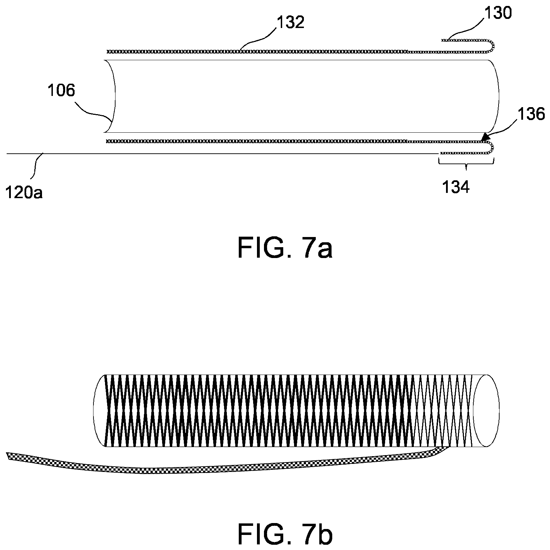

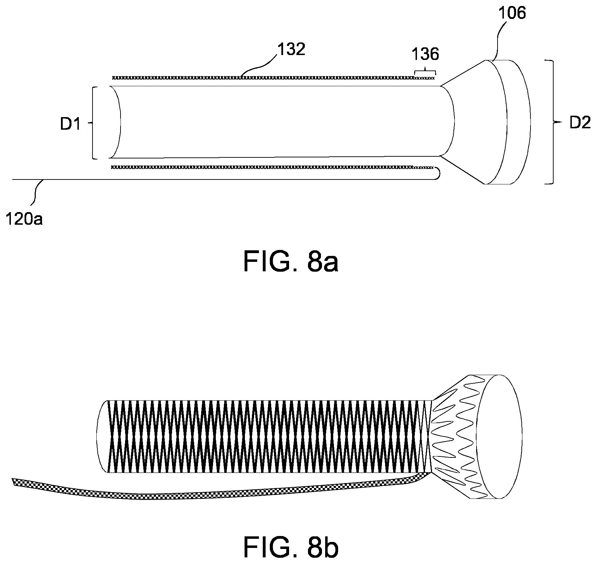

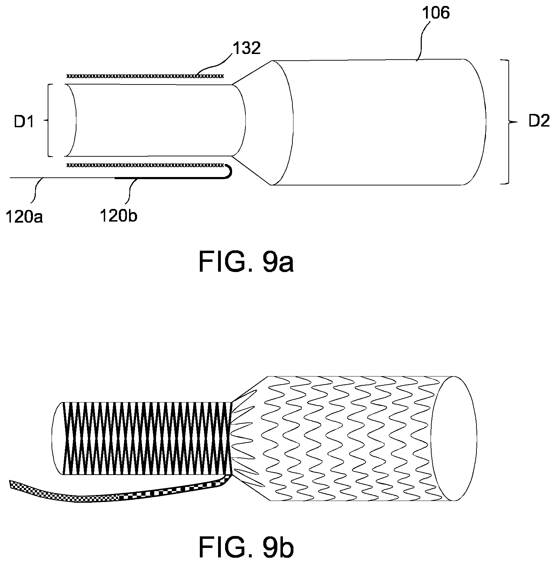

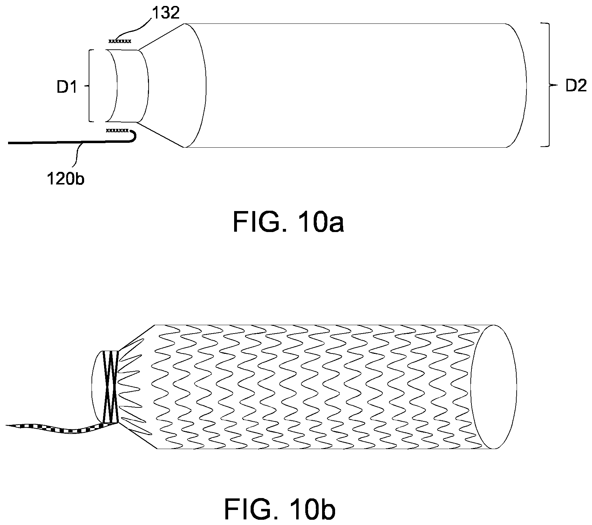

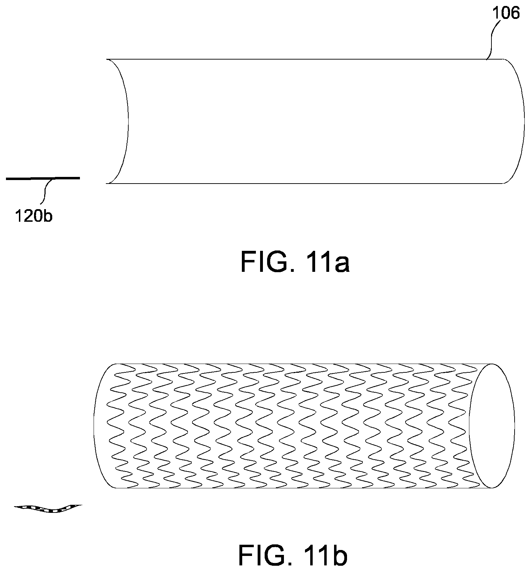

FIGs. 4a-11b show an exemplary embodiment and its deployment. As seen in FIGs. 4a and 4b, the device 106 having a proximal end 110 and a distal end 111 is constrained at a first diameter D1. The device 106 is constrained by a removable constraint 104 including a first segment 130 and a second segment 132. The removable constraint 104 may also be defined by an axially interior layer 150 and an axially exterior layer 152. The axially exterior layer 152 comprises an everted portion 134 of the first segment 130. The axially interior layer 150 comprises the second segment 132 and a non-everted portion 136 of the first segment 130. The device 106 includes a first portion 140 and a second portion 142, with the first portion 140 being along a first length of the longitudinal length of the device 106 at the distal end 111 defined by the non-everted portion 136 of the first segment 130 of the removable constraint 104. The second portion 142 of the device 106 is along a second length of the longitudinal length of the device 106 at the proximal end 110 defined by the second segment 132.

In certain instances, the first segment 130 of the removable constraint 104 is operable to constrain at least the first portion 140 of the device 106, and the second segment 132 of the removable constraint 104 is operable to constrain the second portion 142 of the device 106. The first segment 130 includes a first deployment line 120a and the second segment 132 includes a second deployment line 120b (not shown in FIGs. 4a-11b). The second deployment line 120b of this embodiment is coupled to the first segment 130 of the removable constraint 104.

The everted portion 134 of the of first segment 130 may be disposed circumferentially about the device. In certain instances, the everted portion 134 of the of first segment 130 may be arranged along a length of the device 106 with the deployment line 120 extending from the everted portion 134 at the proximal end 110 of the device 106. As the deployment line 120 is actuated, the first segment 130 will sequentially release from the device 106 starting at the proximal end 110 toward the distal end 111 of the device 106. In a non-limiting example, the first segment 130 may be operable to be deployed as a result of a first deployment force applied to the first deployment line 120a. The second segment 132 may be operable to be deployed as a result of a second deployment force applied to the second deployment line 120b. For reasons discussed in further detail below, the first deployment force may be lower than the second deployment force in this example.

Because the everted portion 134 is positioned radially exterior to the second segment 132 and the non-everted portion 136 of the first segment 130, and as the everted portion 134 is released, the second segment 132 and the non-everted portion 136 of the first segment 130 will be revealed along the length of the device 106. Once the everted portion 134 of the first segment 130 has been released from the device 106, the non-everted portion 136 will begin to sequentially release from the distal end 111 to the proximal end 110 of the device 106. As the non-everted portion 136 is released, the first portion 140 of the device 106 is free to expand. In certain instances, once the non-everted portion 136 of the first segment 130 is fully removed from the device 106, the distal end 111 of the device 106 will be deployed from a first diameter to a second diameter. When this is done in the anatomy (e.g., vasculature) of a patient, the distal end 111 of the device 106 expand and engage with the patient's tissue. Engagement may occur via the outward radial force of the device 106 acting on the patient's tissue. Thus, when the device 106 is deployed in a patient, the second diameter D2 may be the diameter to which the device is capable of expanding while accounting for the resistance that is provided by the tissue of the patient. The device 106 may include engagement devices (e.g., anchors, which are not shown) to engage the device 106 with the patient's tissue, or the radial forces of the device 106 and the tissue may provide for an interference fit.

Once the distal end 111 of the device 106 is deployed and engaging the tissue of the patient, the distal end will act as an anchor to the patient's lumen against relative movement between the device 106 and the patient's lumen during the deployment of the remainder of the device 106. In some examples, because the device 106 is constrained to a relatively small diameter, the device 106 may exert relatively high radial forces. The high radial forces exerted on the removable constraint 104, which may require use of a removable constraint 104 operable to withstand increased radial force without deploying. However, in some situations, increasing the ability of the removable constraint 104 to resist the high radial forces may also increase the deployment force necessary to activate release of the removable constraint 104 from the device 106. In this embodiment, the first segment 130 is operable to require a relatively lower deployment force to release, which allows the first portion 140 of the device 106 to be deployed and to act as an anchor to the patient's lumen against relative movement between the device 106 and the patient's lumen while the second segment 132 is being deployed. Because the first segment 130 is deployed at a lower deployment force, the surgeon may position and deploy the first portion 140 of the device 106 with greater accuracy and / or precision.

As shown in FIGs. 4a-11b, because the first segment is warp-knit and because the first segment 130 sequentially releases beginning or continuing from a position exterior to the second segment 132, unintentional release or "creep" or unraveling of the constraint 104 is reduced because the second segment 132 is configured to apply sufficient constraining force to the device 106 without unintentional deployment. Because it is unlikely for release of the first segment 130 to occur from the "creep" occurring at the interface of the first segment 130 and the first deployment line 120a, the first segment will likely not unintentionally release as unintentional deployment would require one of the strands 108 to break, thus leading to release. Because the removable constraint 104 is configured to release sequentially, unintentional release of the removable constraint 104 at a position other than at the leading edge where the deployment line 120 interfaces with the removable constraint 104 is reduced and unlikely.

Once the first segment 130 has been released and the first portion 140 of the device 106 has been deployed and is acting as an anchor to the patient's lumen, the second segment 132 may be released from the second portion 142 of the device 106. Because the second segment 132 provides increased resistance to the radial expansion of the device 106, the second deployment force may also be increased. Although the second deployment force may be increased, because the first portion 140 of the device 106 is already deployed and engaging the patient's lumen, the second segment 132 may be released with less likelihood of deployment at non-targeted position within the lumen.

The second segment 132 is deployed by applying the second deployment force to or across the second deployment line 120b. As shown in FIGs 4a-11b, the second deployment line 120b may be coupled to or extend from the first segment 130. Thus, the second segment 132 may be deployed by applying the second deployment force to the first deployment line 120a which then translates that same force to the second deployment line 120b. As the second segment 132 continues to release, the second portion 142 of the device 106 is deployed. Once the second segment 132 is fully released, the removable constraint 104 is fully released and may be removed from the patient, through the catheter or otherwise.

### Methods of Manufacture

The disclosure also relates to methods of manufacturing an expandable medical device delivery system. The method may include compressing a device radially inward to a first compressed diameter. A plurality of strands may be knitted to form a first segment of a removable constraint. The plurality of strands may either be knitted directly on the compressed device, or they may be knitted and then installed onto the compressed device. The plurality of strands may be knitted into the first segment of the removable constraint which has a length less than the longitudinal length of the device. A second segment of the removable constraint is also knitted, wherein the second segment may be knitted directly on the compressed device or may be knitted and then installed onto the compressed device.

The methods may include providing a first free end of a first deployment line such that at least a portion of the first deployment line extends away from the removable constraint. The first free end may be operable to deconstruct the removable constraint when tensioned. A second deployment line may also be provided. The second deployment line may extend away from the removable constraint. The method may also comprise coupling the second deployment line to the first segment of the removable constraint. Coupling the second deployment line to the first segment may include fusing, interweaving, or welding.

In some embodiments, the second segment is installed and / or knitted onto the device first. Once the second segment has been installed, the first segment may be installed and / or knitted on the device. When installing and / or knitting the first segment, the method may include everting the first segment.

The methods of manufacturing may also include weakening at least one of the strands comprising the covering member, such that the strand is operable to break. When at least one of the strands breaks, the release of the segment may occur. Thus, in some embodiments, one strand from each of the segments may be weakened in order to facilitate the breaking of a strand from each of the segments. In some embodiments, the weakened strands comprise the deployment lines of the knit rows. In other embodiments, the unweakened strands comprise the deployment lines of the knit rows, such that when the unweakened strands are tensioned, the unweakened strands stay intact and the weakened strands break, which initiates release of the segments. The segments may initially be tensioned in order to provide the first break. Tension may then be applied to the unbroken, unweakened strands in order to continue release of the segments as the segments unravel under tension due to the unraveling of portions of the removable constraint. The removable constraint continues to unravel due to the break in the strand which allows for the removable constraint to unravel or separate. The weakening of the strands may be accomplished by scoring, cutting, treating, or otherwise compromising the strand such that the strands may break under predetermined circumstances such as tensioning.

As part of the manufacturing method, deployment of the removable constraint may be initiated by breaking at least one strand from each of the knit rows. The removable constraint may be deployed to a desired length relative to the device. For example, the removable constraint may be knit to a length longer than the expandable member. The covering member may be activated such that release is initiated. The removable constraint may be partially released until a desired length of the removable constraint is achieved, such as a length where the removable constraint is surrounding the device, as previously described. When a desired length of the segments of the removable constraint is achieved, the deployment may be discontinued until the medical device is prepared for deployment at the target site. It will be understood that the removable constraint may be partially deployed to any desirable length. The partially deployed removable constraint that is constraining the expandable member may then be prepared for packaging, use, installation on a catheter, or any other desired action.

In some embodiments, the method of manufacturing includes performing the step of compressing the device simultaneously with the step of knitting the removable constraint such that the plurality of strands provide a compressive force to the device as the plurality of strands are knitted about the device.

In some embodiments, the removable constraint may be knitted on a mandrel. Once the removable constraint is knitted, and in some embodiments partially deployed, the removable constraint may be removed from the mandrel and applied over a radially compressed implantable medical device. In this embodiment, the step of installing the first segment of the removable constraint onto the radially compressed implantable medical device may include everting a portion of the first segment of the removable constraint back over the radially compressed implantable device. This step may also include concealing the second deployment line between the first segment and the second segment of the removable constraint.

### Various Embodiments of Removable Constraints

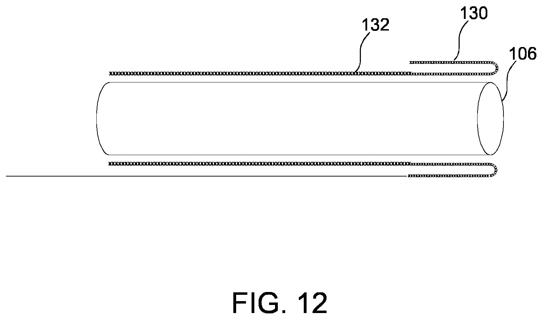

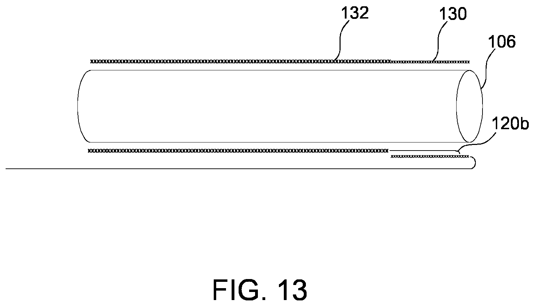

Various embodiments are provided in FIGs. 12-15. For example, FIG. 12 includes a removable constraint having a first and second segment 130, 132. The first segment 130 is partially inverted and the everted portion extends only a portion of the longitudinal length of the device 106. FIG. 13 provides an exemplary embodiment in which the first segment 130 is not inverted. The removable constraint 104 also includes a second deployment line 120b that is coupled to a portion of the first segment 130. The second deployment line 130b is also positioned interior to the first segment 130 such that the second deployment line 120b is revealed after the first segment 130 is released.

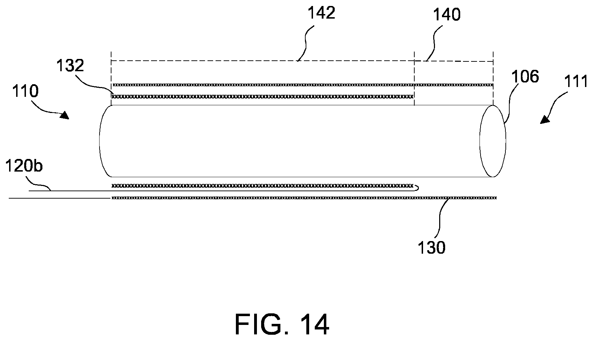

FIG. 14 provides an exemplary embodiment in which the first segment 130 is disposed coaxially with the second segment 132, wherein the second segment 132 is positioned interior to the first segment 130. The first segment 130 is operable to release from the proximal end 110, although it is within the scope of this disclosure that the first segment can release from the distal end 111. The first segment 130 may be non-everted and may cover or conceal at least a portion of the second deployment line 120b. The first portion 140 of the device 106 may deploy when the first segment 130 is released from the device 106 and provide an anchoring portion. The second segment 132 may then be released from the device 106, thus causing the full deployment of the device 106.

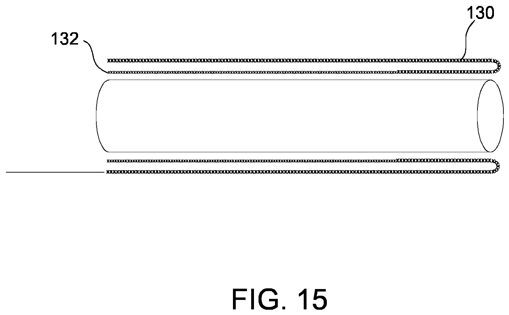

FIG. 15 provides an exemplary embodiment in which various properties of the first and second segments 130, 132 of the removable constraint 104 may be varied. For example, in this embodiment the first segment 130 may be operable to resist greater expansion forces than the second segment 132. This may be a result of the strand properties implemented in the various embodiments. However, FIG. 15 is not to be construed as exhibiting and implementing any one set strand properties to the exclusion of others. As previously discussed, the various properties may be implemented for various reasons.

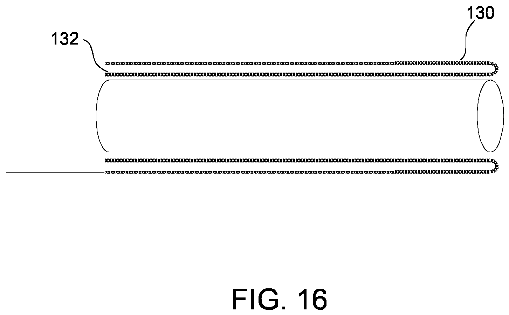

FIG. 16 provides an exemplary embodiment in which various properties of the first and second segments 130, 132 of the removable constraint may be varied and where the second segment 132 surrounds the device from the first end of the device to the second end of the device. The second segment 132 everts at one end of the device such that the everted portion of the second segment 132 surrounds a portion of the non-everted portion of the second segment 132. The first segment 130 surrounds the remainder of the non-everted portion of the second segment 132 that is not surrounded by the everted portion of the second segment 132.

The invention of this application has been described above both generically and with regard to specific embodiments. It will be apparent to those skilled in the art that various modifications and variations can be made in the embodiments without departing from the scope of the disclosure. Thus, it is intended that the embodiments cover the modifications and variations of this invention provided they come within the scope of the appended claims and their equivalents.

The present disclosure is also described by the following numbered clauses:
Clause 1: An implantable medical device comprising:
   an expandable device having a longitudinal length and operable to be compressed to a first diameter and deployed to a second diameter, wherein the second diameter is greater than the first diameter, and wherein a first portion that is less than the longitudinal length of the expandable device and a second portion that is less than the longitudinal length of the expandable device are defined on the expandable device;
   a first constraining member operable to constrain the first portion of the expandable device at the first diameter;
   a second constraining member operable to constrain the second portion of the expandable device at the first diameter.
Clause 2: The implantable medical device of clause 1, wherein the first constraining member defines a first release zone and the second constraining member defines a second release zone.
Clause 3: The implantable medical device of clause 2, wherein the first constraining member further comprises a first deployment line, wherein the first deployment line is configured to release the first constraining member along the first release zone by tensioning the first deployment line, and
   wherein the second constraining member further comprises a second deployment line, wherein the second deployment line is configured to release the second constraining member along the second release zone by tensioning the second deployment line.
Clause 4: The implantable medical device of clause 3, wherein the first release zone is configured to release when a first tension force is applied across the first deployment line,
   wherein the second release zone is configured to release when a second tension force is applied across the second deployment line, and
   wherein the first tension force is higher than the second tension force.
Clause 5: The implantable medical device of clause 3, wherein the first release zone is configured to release when a first tension force is applied across the first deployment line,
   wherein the second release zone is configured to release when a second tension force is applied across the second deployment line, and
   wherein the first tension force is lower than the second tension force.
Clause 6. The implantable medical device of any of the preceding clauses, wherein the second constraining member extends over at least part of the first portion of the expandable member.
Clause 7: The implantable medical device of clause 6, wherein the second constraining member is positioned between the first constraining member and the expandable member where the second constraining member extends over at least part of the first portion of the expandable member.
Clause 8: The implantable medical device of clause 6, wherein the second constraining member is disposed exterior to the first constraining member and the expandable member where the second constraining member extends over at least part of the first portion of the expandable member.
Clause 9: The implantable medical device of any of the preceding clauses, wherein the second constraining member includes an everted portion.
Clause 10: The implantable medical device of clause 9, wherein the everted portion of the second constraining member is disposed about at least part of the first portion expandable member and the first constraining member.
Clause 11: The implantable medical device of clause 10, wherein the everted portion of the second constraining member is disposed along the longitudinal length of the expandable member.
Clause 12: The implantable medical device of any of the preceding clauses, wherein the first constraining member comprises a first knit sleeve comprising a first plurality of strands.
Clause 13: The implantable medical device of any of the preceding clauses, wherein the second constraining member comprises a second knit sleeve comprising a second plurality of strands.
Clause 14: The implantable medical device of clause 12 or 13, wherein the first knit sleeve and the second knit sleeve are knitted.
Clause 15: The implantable medical device of any of the preceding clauses, wherein the first constraining member and the second constraining member are warp knit.
Clause 16: The implantable medical device of any of the preceding clauses, wherein the first constraining member is operable to release after the second constraining member has been released.
Clause 17: The implantable medical device of any one of clauses 3-16, wherein the first deployment line is engaged with the second constraining member such that when the second constraining member is released, the first deployment line is accessible to activate release of the first constraining member.
Clause 18: The implantable medical device of clause 17, wherein the first deployment line is coupled to the second constraining member such that when the second constraining member has translated away from the expandable member, the first deployment line is tensioned and activates release of the first constraining member.
Clause 19: The implantable medical device of any one of clause 12-18, wherein the first plurality of strands and second plurality of strands include matching strand properties, including any one of strand thickness, strand denier, strand coefficient of friction, strand material, strand coatings, strand treatments, strand stiffness, or any combination thereof.
Clause 20: The implantable medical device of any one of clauses 12-18, wherein the first plurality of strands and second plurality of strands include differing strand properties, including any one of strand thickness, strand denier, strand coefficient of friction, strand material, strand coatings, strand treatments, strand stiffness, or any combination thereof.
Clause 21: The implantable medical device of any one of clauses 12-20, wherein the second plurality of strands extend from the first plurality of strands.
Clause 22: An implantable medical device comprising:
   an expandable member having a proximal end and a distal end, wherein the expandable member has a longitudinal length defined between the proximal end and the distal end;
   a first constraining member disposed about the expandable member at a first portion that is less than all of the longitudinal length of the expandable member, wherein the first constraining member maintains the expandable member at the first portion at a constrained diameter; and
   a second constraining member including a first segment and an everted segment, wherein the first segment of the second constraining member is disposed about the expandable member at a stabilizing portion that is a remainder of the longitudinal length where the first constraining member is not disposed about the expandable member, wherein the second constraining member maintains the expandable member at the stabilizing portion at the constrained diameter,
   wherein the everted segment of the second constraining member is disposed about the first segment of the second constraining member and the stabilizing portion of the expandable member.
Clause 23: The implantable medical device of clause 22, wherein the first constraining member further comprises a first release zone and a first deployment line, and wherein the second constraining member further comprises a second release zone and a second deployment line.
Clause 24: The implantable medical device of clause 23, wherein the first deployment line is operable to release the first release zone when at least a first deployment force is applied to the first deployment line, and wherein the second deployment line is operable to release the second release zone when at least a second deployment force is applied to the second deployment line.
Clause 25: The implantable medical device of clause 24, wherein the first deployment force is greater than the second deployment force.
Clause 26: The implantable medical device of clause 24, wherein the first deployment force is less than the second deployment force.
Clause 27: The implantable medical device of any one of clauses 22-26, wherein the first constraining member comprises a first plurality of warp-knit strands.
Clause 28: The implantable medical device of any one of clauses 22-27, wherein the second constraining member comprises a second plurality of warp-knit strands.
Clause 29: The implantable medical device of any one of clauses 22-28, wherein the everted segment of the second constraining member is disposed about the first constraining member.
Clause 30: The implantable medical device of any one of clauses 22-29, wherein the stabilizing portion of the expandable member is operable to deploy to a second diameter when the second constraining member is removed from the expandable member.
Clause 31: The implantable medical device of any one of clauses 23-30, wherein the first deployment line is engaged with the second constraining member such that first constraining member is operable to be released after the second constraining member has been released and the stabilizing portion of the expandable member has been deployed.
Clause 32: The implantable medical device of any one of clauses 22-31, wherein the first portion of the expandable member extends to the proximal end of the expandable member.
Clause 33: The implantable medical device of any one of clauses 22-32, wherein the stabilizing portion of the expandable member extends to the distal end of the expandable member.
Clause 34: The implantable medical device of any one of clauses 22-33, wherein the second sheath is configured to release such that the proximal end of the expandable member is deployed first.
Clause 35: The implantable medical device of any one of clauses 22-34, wherein the first sheath is configured to release after the second sheath is released, such that the proximal end of the expandable member is deployed subsequent to the distal end being deployed.
Clause 36: A method of manufacturing an implantable medical device, comprising:
   compressing an expandable member radially inward;
   knitting a first plurality of strands to form a first constraining member with a first deployment line;
   installing the first constraining member on a first portion of the expandable member;
   knitting a second plurality of strands to form a second constraining member;
   installing the second constraining member on a second portion of the expandable member; and
   everting a segment of the second constraining member back over the second portion of the expandable member.
Clause 37: The method of clause 36, further comprising initiating release of a first release zone of the first constraining member and a second release zone of the second constraining member.
Clause 38: The method of clause 37, further comprising partially releasing the first release zone until a first end of the first release zone is positioned proximate a proximal end of the expandable member.
Clause 39: The method of clause 38, further comprising partially releasing the second release zone until a second end of the second release zone is positioned proximate the proximal end of the expandable member.
Clause 40: A method of deploying an implantable medical device, comprising:
   positioning an expandable member in a lumen of a patient, wherein a first portion of the implantable medical device is constrained by a first constraining member in a compressed configuration and a second portion of the expandable member is constrained by a second constraining member in the compressed configuration;
   removing the second constraining member from the second portion of the expandable member;
   removing the first constraining member from the first portion of the expandable member.
Clause 41: The method of clause 40, further comprising:
   retaining a free end of a second deployment line remote from the expandable medical device, wherein the second deployment line is engaged with a release zone of the second constraining member that is configured to disengage the second constraining member from the expandable member; and
   applying sufficient force to the free end of the second deployment line to activate the second release zone.
Clause 42: The method of clause 41, further comprising applying sufficient force to a free end of a first deployment line to activate a release zone of the first constraining member, wherein the first deployment line is engaged with the release zone of the first constraining member, wherein the release zone of the first constraining member is configured to disengage the first constraining member from the expandable member.

## Claims

1. An implantable medical device comprising:
an expandable member having a proximal end and a distal end, wherein the expandable member has a longitudinal length defined between the proximal end and the distal end;
a first constraining member disposed about the expandable member at a first portion that is less than all of the longitudinal length of the expandable member, wherein the first constraining member maintains the expandable member at the first portion at a constrained diameter; and
a second constraining member including a first segment and an everted segment, wherein the first segment of the second constraining member is disposed about the expandable member at a stabilizing portion that is a remainder of the longitudinal length where the first constraining member is not disposed about the expandable member, wherein the second constraining member maintains the expandable member at the stabilizing portion at the constrained diameter,
wherein the everted segment of the second constraining member is disposed about the first segment of the second constraining member and the stabilizing portion of the expandable member.

2. The implantable medical device of claim 1, wherein the first constraining member further comprises a first release zone and a first deployment line, and wherein the second constraining member further comprises a second release zone and a second deployment line.

3. The implantable medical device of claim 2, wherein the first deployment line is operable to release the first release zone when at least a first deployment force is applied to the first deployment line, and wherein the second deployment line is operable to release the second release zone when at least a second deployment force is applied to the second deployment line.

4. The implantable medical device of claim 3, wherein the first deployment force is greater than the second deployment force.

5. The implantable medical device of claim 3, wherein the first deployment force is less than the second deployment force.

6. The implantable medical device of any one of claims 1-5, wherein the first constraining member comprises a first plurality of warp-knit strands.

7. The implantable medical device of any one of claims 1-6, wherein the second constraining member comprises a second plurality of warp-knit strands.

8. The implantable medical device of any one of claims 1-7, wherein the everted segment of the second constraining member is disposed about the first constraining member.

9. The implantable medical device of any one of claims 1-8, wherein the stabilizing portion of the expandable member is operable to deploy to a second diameter when the second constraining member is removed from the expandable member.

10. The implantable medical device of any one of claims 2-9, wherein the first deployment line is engaged with the second constraining member such that first constraining member is operable to be released after the second constraining member has been released and the stabilizing portion of the expandable member has been deployed.

11. The implantable medical device of any one of claims 1-10, wherein the first portion of the expandable member extends to the proximal end of the expandable member.

12. The implantable medical device of any one of claims 1-11, wherein the stabilizing portion of the expandable member extends to the distal end of the expandable member.

13. The implantable medical device of any one of claims 1-12, wherein the second sheath is configured to release such that the proximal end of the expandable member is deployed first.

14. The implantable medical device of any one of claims 1-13, wherein the first sheath is configured to release after the second sheath is released, such that the proximal end of the expandable member is deployed subsequent to the distal end being deployed.
